# EUROPÄISCHE PATENTSCHRIFT

(11) **EP 0 496 845 B1**
(45) Veröffentlichungstag und Bekanntmachung des Hinweises auf die Patenterteilung: **08.01.1997**
(21) Anmeldenummer: 91913340.5
(22) Anmeldetag: 30.07.1991
(51) Int. Cl.: C12P 33/02

(54) **VERFAHREN ZUR HERSTELLUNG VON 4-PREGNEN-3,20-DION UND SEINEN DERIVATEN**
PROCESS FOR PRODUCING 4-PREGNENE-3,20-DIONE AND ITS DERIVATIVES
PROCEDE DE PRODUCTION DE 4-PREGNEN-3,20-DIONE ET DE SES DERIVES

(30) Priorität: 18.08.1990 DE 4026464
(43) Veröffentlichungstag der Anmeldung: 05.08.1992
(73) Patentinhaber: SCHERING AKTIENGESELLSCHAFT, 13342 Berlin (DE)
(72) Erfinder: WEBER, Alfred, D-1000 Berlin 37 (DE); KENNECKE, Mario, D-1000 Berlin 33 (DE)
(86) Internationale Anmeldenummer: DE9100620
(87) Internationale Veröffentlichungsnummer: WO9203571

(56) Entgegenhaltungen:
- EP-A- 0 108 231
- WO-A-87/05940
- US-A- 3 759 791
- Applied Microbiology 23, 1, 1972, 72 - 77
- Steroids 11, 1968, 401 - 413

## Beschreibung

Die Erfindung betrifft ein Verfahren zur Herstellung von 4-Pregnen-3,20-dion und seinen Derivaten der allgemeinen Formel I 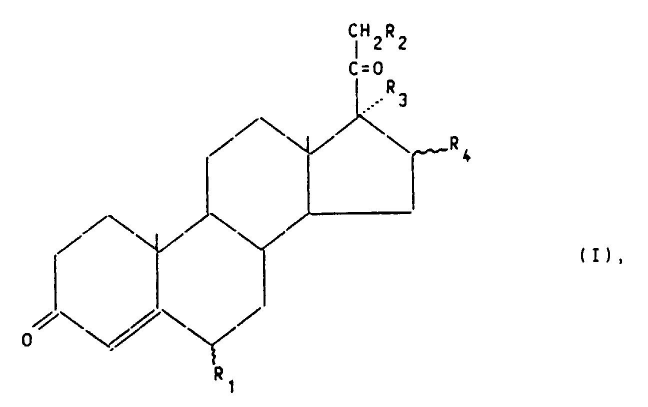 worin
- R₁: ein Wasserstoffatom. ein Fluoratom oder eine Methylgruppe bedeutet,
- R₂: ein Wasserstoffatom oder eine Hydroxygruppe darstellt, und
- R₃ und R₄: gemeinsam eine Kohlenstoff-Kohlenstoffbindung symbolisieren oder
- R₃: ein Wasserstoffatom. eine Hydroxygruppe oder eine Alkanoyloxygruppe mit bis zu 6 Kohlenstoffatomen darstellt und
- R₄: ein Wasserstoffatom oder eine Methylgruppe bedeutet,
dadurch gekennzeichnet,
daß man ein Pregnan-Derivat der allgemeinen Formel II 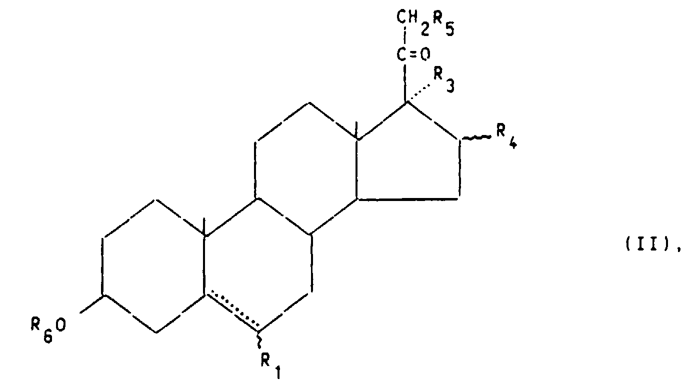 worin R₁, R₃ und R₄ die obengenannte Bedeutung besitzen,
..... eine Einfachbindung oder eine Doppelbindung symbolisiert,
- R₅: ein Wasserstoffatom, eine Hydroxygruppe oder eine Alkanoyloxygruppe mit maximal 6 Kohlenstoffatomen darstellt und
- R₆: ein Wasserstoffatom oder eine Alkanoylgruppe mit maximal 6 Kohlenstoffatomen bedeutet, mit einer Bakterienkultur der Spezies Mycobacterium spec. NRRL 8-3805 fermentiert.

Es ist bekannt, daß man Verbindungen der Formel I aus im B-Ring ungesättigten Verbindungen der Formel II durch Fermentation mit Mikroorganismen herstellen kann. So wird beispielsweise in der EP-A 0 108 231 die Herstellung von 3-Keto-Δ⁴-steroiden durch Fermentation mit immobilisierten Flarobacterium dehydrogenans Biokatalysatoren beschrieben.

In der EP-A 0 002 535 wird ein Verfahren offenbart, 3-Keto-Δ⁴-steroide durch Umsetzung von Zoo- oder Phytosterium mit Mycobacteriums spec. NRRL-B 3805 in Gegenward von Borationen herzustellen.

Diese Reaktionen eigenen sich aber nicht zur Umsetzung von im B-Ring gesättigten Steroiden der allgemeinen Formel IIa zu Verbindungen der allgemeinen Formel I.

Die vorliegende Erfindung ist von besonderer Bedeutung für die Partialsynthese pharmakologisch wirksamer Pregnan-Derivate aus den in der Natur weit verbreiteten Steroidsapogeninen, Smilagenin und Sarsasapogenin. Es ist seit langem bekannt, daß man diese Sapogenine relativ einfach zu 3β-Hydroxy-5β-3β-Hydroxy-5β-pregn-16-en-20-on beziehungsweise dessen 3-Acetat abbauen kann (US-A 3,475,464 und Canadian Journ. of Chem., 46, 1968, 733 f). Bei diesem Verbindungen kann man mittels an sich bekannter Methoden in der 16-Position eine Methylgruppe und/ oder in der 17a- und/oder 21-Position eine Methylgruppe und/oder in der 17α- und/oder 21-Position eine Hydroxygruppe oder Acyloxygruppe einführen, oder man kann die 16-Doppelbindung dieser Substanzen hydrieren. (John Fried und John A. Edwards "Organic Reactions in Steroid Chemistry"; van Nostrand Reinhold Comp. New York, etc. Vol. 1 1972, p 125 ff. Vol 2, 1972, p 075f und Vol 2, 1972, p 162f und 176f).

Die so dargestellten Pregnan-Derivate der allgemeinen Formel IIa 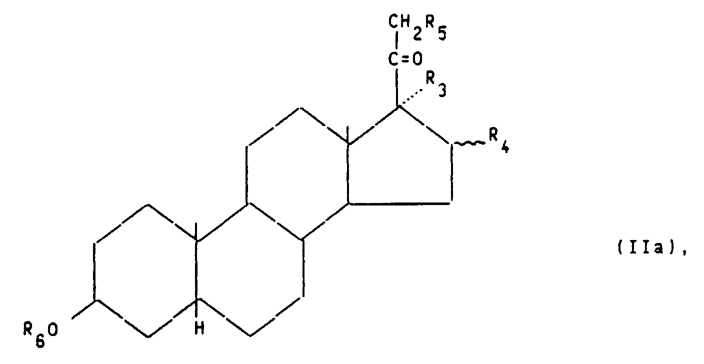 worin R₃, R₄ und R₅ und R₆ die bereits genannte Bedeutung besitzen, werden nach dem bekannnten Stand der Technik in einem mehrstufigen chemischen Verfahren, das mittels umweltbelastenden Agentien durchgeführt wird, in die entsprechenden 3-Oxo-Δ⁴-steroide überführt (US-A 3,475,464).

Demgegenüber ermöglicht es das erfindungsgemäße Verfahren, diese Verbindungen in einem einstufigen Verfahren unter Erzielung guter Ausbeuten in die entsprechenden 3-Oxo-Δ⁴-steroide zu überführen . Das dies möglich ist, ist für den Fachmann sehr überraschend, denn es ist bekannt, daß der bei diesem Verfahren verwendete Mikroorganismus üblicherweise die Seitenketten von Steroiden zu den entsprechenden 17-Oxosteroiden abbaut. (US-A 4,179,336).

Das erfindungsgemäße Verfahren wird unter den gleichen Fermentationsbedingungen durchgeführt, welche man auch bei den bekannten mikrobiologischen Umwandlungen von Substraten mit diesen Bakterienkulturen anwendet.

Unter den für diese Mikroorganismen üblicherweise verwendeten Kulturbedingungen werden in einem geeigneten Nährmedium unter Belüften Submerskulturen angezüchtet. Dann setzt man den Kulturen das Substrat (in einem geeigneten Lösungsmittel gelöst oder in emulgierter Form) zu und fermentiert, bis eine maximale Substratumwandlung erreicht ist.

Geeignete Substratlösungsmittel sind beispielsweise Methanol, Ethanol, Glykolmonomethylether, Dimethylformamid oder Dimethylsulfoxyd. Die Emulgierung des Substrats kann beispielsweise bewirkt werden, indem man dieses in mikronisierter Form oder in einem mit Wasser mischbaren Lösungsmittel (wie Methanol, Ethanol. Aceton, Glykolmonomethylether. Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Ethylenoxyaddukte oder Fettsäureester von Polyglykolen. Als geeignete Emulgatoren seien die handelsüblichen Netzmittel Tegin®, Tween® und Span® beispielsmäßig genannt.

Die optimale Substratkonzentration, Substratzugabezeit und Fermentationsdauer ist von der Art des verwendeten Substrates und Mikroorganismus und den Fermentationsbedingungen abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Das erfindungsgemäße Verfahren kann auch unter Verwendung anderer PregnanDerivate der allgemeinen Formel II als solches der Formel IIa durchgeführt werden, dies bringt aber nach bisheriger Kenntnis gegenüber den bekannten mikrobiologischen Verfahren kaum Vorzüge.

Die nachfolgenden Ausführungsbeispiele dienen zur näheren Erläuterung des erfindungsgemäßen Verfahrens.

### Beispiel 1

a) Ein 2 1 Erlenmeyer mit 500 ml sterilem Nährmedium enthaltend
   1 % Hefeextrakt
   0,45 % Na₂HPO₄
   0,34 % KH₂PO₄
   0,2 % Tween 80
   -eingestellt auf pH 6,7-
   wird mit einer Abschwemmung einer Trockenkultur von Mycobacterium spec. NRRL B-3805 beimpft und 3 Tage mit 180 Umdrehungen pro Minute bei 30° C geschüttelt.
b) 10 g 3β-Acetoxy-5β-16-pregnen-20-on (Canad. J. of Chem., 46, 1968, 734 ff) werden in einer Kugelmühle (PE 075, Fa. Netzsch, DE-Selb/Bayern) mit Korundkugeln auf einer Teilchengröße von ca. 1µ vermahlen und mit destilliertem Wasser auf ein Endvolumen von 500 ml eingestellt.
c) 50 Erlenmeyer (100 ml) mit jeweils 20 ml sterilem Nährmedium enthaltend
   2,5 Z Cornsteep liquor
   0,25 % Sojamehl
   0,3 % (NH₄)₂HPO₄
   0,25 % Tween 80
   - eingestellt auf pH 6,5 -
   werden mit jeweils 1 ml der Mycobacterium-spec.-Anzuchtskultur beimpft. Anschließend werden jeweils 3 ml der unter b) hergestellten Mahlsuspension zugesetzt, was 0,06 g 3β-Acetoxy-5β-16-pregnen-20-on entspricht, und 120 Stunden bei 30° C auf einem Rotationsschüttler mit 220 Umdrehungen pro Minute fermentiert.

Die vereinigten Kulturen werden mit Methylisobutylketon extrahiert, mit 100 g Aktivkohle versetzt und über einem Faltenfilter filtriert. Das Filtrat wird anschließend bei max. 50° C im Rotationsverdampfer unter Vakuum eingeengt und am Aluminiumoxid chromatographiert.

Man erhält so 0.7 g 4,16-Pregandien-3,20-dion, welches nach HPLC mit einer authentischen Probe identisch ist.

### Beispiel 2

a) 10 g 3β-Acetoxy-5β-16-pregnen-20-on (Canad. J. of Chem., 46, 1968, 734 ff) werden vermahlen wie in Beispiel 1 b beschrieben und mit destilliertem Wasser auf 500 ml eingestellt.
b) Unter den Bedingungen des Beispiels 1 c werden in 50 Erlenmeyerkolben mit je 100 ml Fermentationskultur je 3 ml der obigen Suspension zugesetzt, fermentiert und aufbereitet.

Man erhält so 0,75 g 4-Pregnen-3,20-dion, welches nach HPLC mit einer authentischen Probe identisch ist.

### Beispiel 3

a) 10 g 3β-Hydroxy-5β-pregnan-20-on (Canad. J. of Chem., 46, 1968, 734 ff) werden wie im Beispiel 1 b beschrieben vermahlen und mit 500 ml destilliertem Wasser auf 500 ml eingestellt.
b) Unter den Bedingungen des Beispiels 1 c werden in 50 Erlenmeyerkolben mit je 100 ml Fermentationskultur je 3 ml der obigen Suspension zugesetzt, fermentiert und aufbereitet.

Man erhält so 1.0 g 4-Pregnen-3,20-dion, welches nach HPLC mit einer authentischen Probe identisch ist.

### Beispiel 4

a) 10 g 21-Acetoxy-3β-hydroxy-methyl-5β-pregnan-20-on (DE-8 22 57 132) werden vermahlen, wie in Beispiel 1 b beschrieben und mit destilliertem Wasser auf 500 ml eingestellt.
b) Unter den Bedingungen des Beispiels 1 c werden in 50 Erlenmyerkolben mit je 100 ml Fermentationskultur je 3 ml der obigen Suspension zugesetzt, fermentiert und aufbereitet.

Man erhält so 0,2 g 21-Hydroxy-16α-methyl-4-pregnen-3,20-dion, welches nach HPLC mit einer authentischen Probe identisch ist.

## Patentansprüche

1. Verfahren zur Herstellung von 4-Pregnen-3,20-dion und seinen Derivaten der allgemeinen Formel I 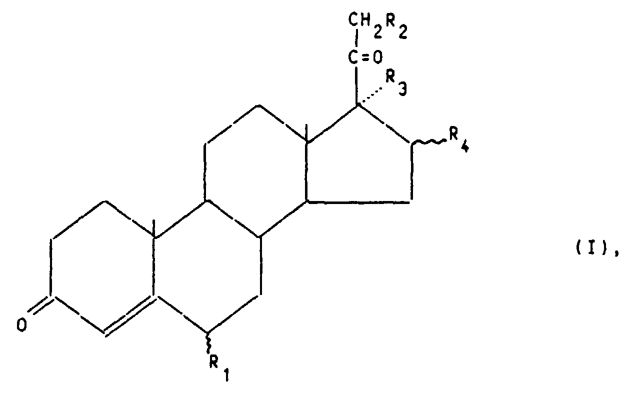 worin
R₁ ein Wasserstoffatom, ein Fluoratom oder eine Methylgruppe bedeutet,
R₂ ein Wasserstoffatom oder eine Hydroxygruppe darstellt, und
R₃ und R₄ gemeinsam eine Kohlenstoff-Kohlenstoffbindung symbolisieren oder
R₃ ein Wasserstoffatom, eine Hydroxygruppe oder eine Alkanoyloxygruppe mit bis zu 6 Kohlenstoffatomen darstellt und
R₄ ein Wasserstoffatom oder eine Methylgruppe bedeutet.
dadurch gekennzeichnet,
daß man ein Pregnan-Derivat der allgemeinen Formel II 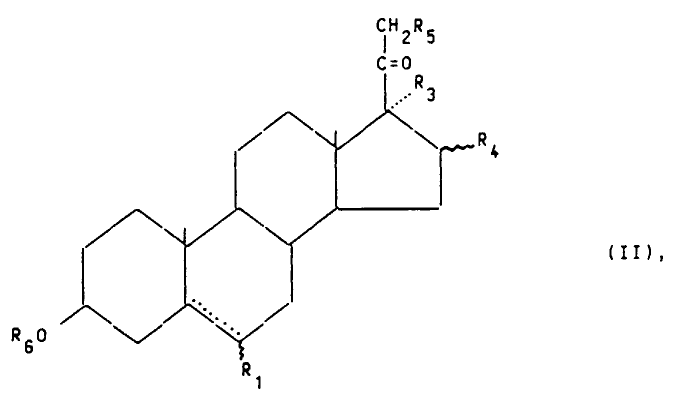 worin R₁, R₃ und R₄ die obengenannte Bedeutung besitzen,
..... eine Einfachbindung oder eine Doppelbindung symbolisiert,
R₅ ein Wasserstoffatom, eine Hydroxygruppe oder eine Alkanoyloxygruppe mit maximal 6 Kohlenstoffatomen darstellt und
R₆ ein Wasserstoffatom oder eine Alkanoylgruppe mit maximal 6 Kohlenstoffatomen bedeutet, mit einer Bakterienkultur der Spezies Mycobacterium spec. NRRL 8-3805 fermentiert.

## Claims

1. Process for the production of 4-pregnene-3,20-dione and its derivatives of general formula I 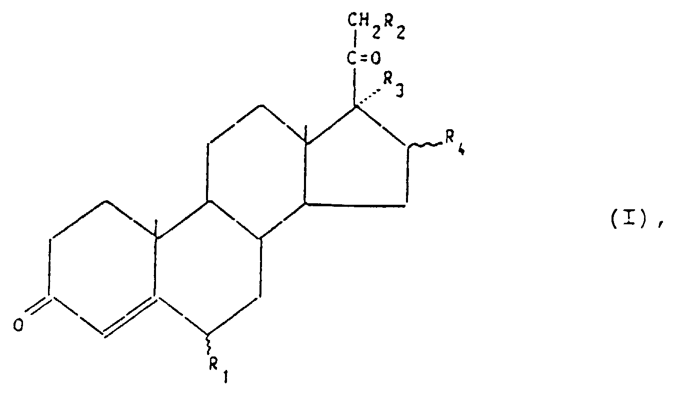 in which
R₁ means a hydrogen atom, a fluorine atom or a methyl group,
R₂ represents a hydrogen atom or a hydroxy group, and
R₃ and R₄ together symbolize a carbon-carbon bond or
R₃ represents a hydrogen atom, a hydroxy group or an alkanoyloxy group with up to 6 carbon atoms and
R₄ means a hydrogen atom or a methyl group,
characterized in that
a pregnane derivative of general formula II 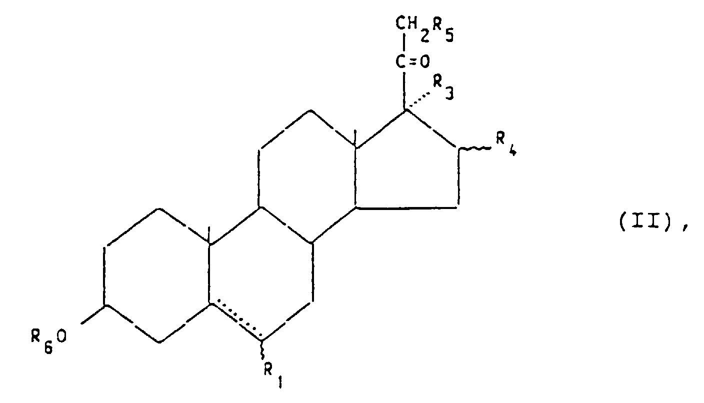 in which R₁, R₃ and R₄ have the above-mentioned meaning,
..... symbolizes a single bond or a double bond,
R₅ represents a hydrogen atom, a hydroxy group or an alkanoyloxy group with at most 6 carbon atoms and
R₆ means a hydrogen atom or an alkanoyl group with at most 6 carbon atoms, is fermented with a bacterial culture of species Mycobacterium spec. NRRL B-3805.

## Revendications

1. Procédé pour la préparation de 4-prégnène-3,20-dione et de ses dérivés de formule générale I 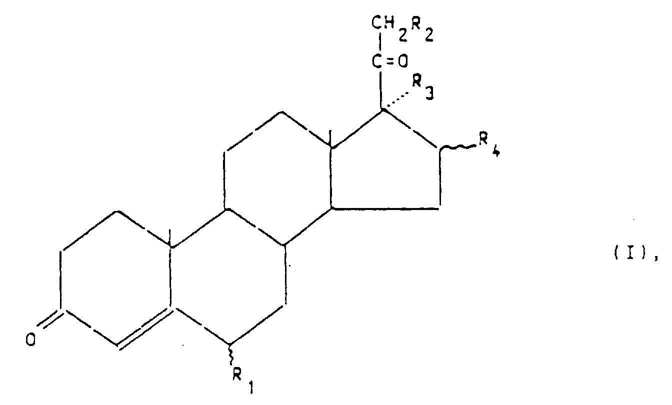 dans laquelle
R₁ représente un atome d'hydrogène, un atome de fluor ou un groupe méthyle,
R₂ représente un atome d'hydrogène ou un groupe hydroxyle, et
R₃ et R₄ ensemble représentent une liaison carbone-carbone ou
R₃ représente un atome d'hydrogène, un groupe hydroxyle ou un groupe alcanoyloxy avec jusqu'à 6 atomes de carbone et
R₄ représente un atome d'hydrogène ou un groupe méthyle,
caractérisé en ce qu'on fermente un dérivé de prégnane de formule générale II 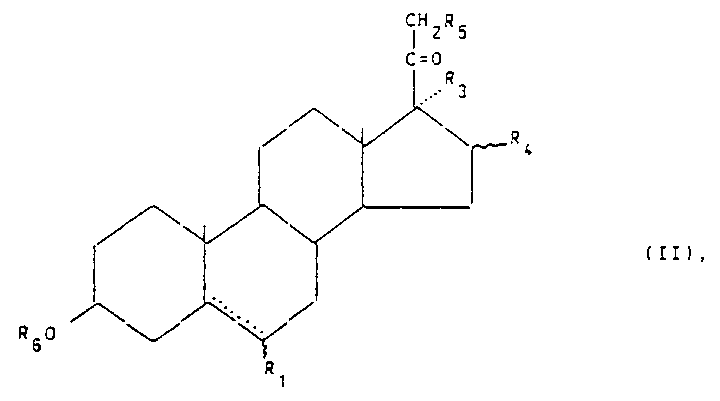 dans laquelle R₁, R₂ et R₃ ont la signification donnée ci-dessus,
.... représente une simple liaison ou une double liaison,
R₅ représente un atome d'hydrogène, un groupe hydroxyle ou un groupe alcanoyloxy avec 6 atomes de carbone au maximum et
R₆ représente un atome d'hydrogène ou un groupe alcanoyle avec 6 atomes de carbone au maximum,
avec une culture de bactéries de l'espèce Mycobacterium spec. NRRL B-3805.
